(19) Europäisches Patentamt
European Patent Office
Office européen des brevets

(11) **EP 4 043 864 A1**

(12) **EUROPEAN PATENT APPLICATION**
published in accordance with Art. 153(4) EPC

(43) Date of publication:
**17.08.2022 Bulletin 2022/33**

(21) Application number: **20883205.5**

(22) Date of filing: **02.10.2020**

(51) International Patent Classification (IPC):
**G01N 21/17** (2006.01)    **C12N 5/071** (2010.01)
**C12Q 1/04** (2006.01)

(52) Cooperative Patent Classification (CPC):
**C12Q 1/04; G01N 21/17**

(86) International application number:
**PCT/JP2020/037590**

(87) International publication number:
**WO 2021/085033 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2019 JP 2019195670**

(71) Applicant: **FUJIFILM Corporation**
**Tokyo 106-8620 (JP)**

(72) Inventors:
• **MURAKAMI, Yuta**
  **Ashigarakami-gun, Kanagawa 258-8577 (JP)**
• **OSAKI, Ryusuke**
  **Ashigarakami-gun, Kanagawa 258-8577 (JP)**
• **ONOZAWA, Sho**
  **Ashigarakami-gun, Kanagawa 258-8577 (JP)**
• **NAKAMURA, Sohichiro**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR SORTING PLURIPOTENT STEM CELLS, METHOD FOR PREDICTING DIFFERENTIATION INDUCTION RESULTS, AND METHOD FOR MANUFACTURING CELL PRODUCT**

(57)    There is provided a method including generating a phase contrast image of an aggregate of a pluripotent stem cell from a hologram in which the aggregates is captured; deriving a phase contrast amount density obtained by dividing a total phase contrast amount which is a value obtained by integrating a phase contrast amount of each of a plurality of pixels that constitute the phase contrast image, by a volume of the aggregate; and sorting the pluripotent stem cell based on the phase contrast amount density.

FIG. 8

**Description**

BACKGROUND OF THE INVENTION

1. Field of the Invention

The disclosed technology relates to a sorting method for a pluripotent stem cell, a prediction method for a differentiation induction result, and a production method for a cell.

2. Description of the Related Art

The following technology is known as a technology for determining a differentiation degree, which indicates a degree of differentiation of a pluripotent stem cell. For example, JP2015-146747A discloses that a cell is observed using a phase contrast microscope to obtain an optical path length N in the inside of the cell nucleus region and an optical path length C in the outside of the cell nucleus region, and a degree of differentiation of the cell is determined based on the ratio C/N, which is a ratio of the optical path length N to the optical path length.

Further, the following technology is known as a technology for determining the quality of induced pluripotent stem cells (iPS cells). For example, JP2018-000048A discloses an evaluation supporting method including a step of calculating a phase distribution of iPS cells from an image signal of a biological specimen of which an image is captured with a microscope that converts a phase distribution into an image intensity distribution, a step of extracting a region having a phase amount equal to or larger than a specific phase amount from the phase distribution, and a step of creating evaluation information serving as an indicator for evaluating the state of the iPS cells and presenting the evaluation information, using the region having a phase amount equal to or larger than a specific phase amount.

Furthermore, there is known a technology for determining whether pluripotent stem cells under being cultured maintain an undifferentiated state or are in a state deviated from the undifferentiated state. For example, WO2018/158901A discloses a cell analysis method characterized by executing a cell region extraction step of extracting a cell region in which cells are present in a phase image of a cell to be analyzed obtained from a hologram obtained with a holographic microscope; a background value acquisition step of calculating a background value based on phase values at a plurality of positions in a region other than the cell region in the phase image; an intracellular phase value acquisition step of obtaining a intracellular phase value based on phase values at a plurality of positions within a measurement target range between a contour line of a cell in the cell region and a virtual line inwardly spaced apart from the contour line by a predetermined distance; and a cell state determination step of determining whether the cell to be analyzed is in an undifferentiated state or a state deviated from the undifferentiated state, based on the difference between the phase value obtained in the intracellular phase value acquisition step and the background value.

SUMMARY OF THE INVENTION

Pluripotent stem cells such as an embryonic stem cell (an ES cell) and an iPS cell have an ability to differentiate into various kinds of cells, and thus they can be applied to drug discovery and regenerative medicine. For example, a cell product that is used in regenerative medicine is obtained by culturing and proliferating an iPS cell and carrying out a differentiation induction treatment for differentiating it into a target cell.

In the process of establishing an iPS cell, there is generally a step of separating a cell group into which reprogramming genes have been introduced into single cells and culturing each of the single cells thereby being cloned. Since obtained clones differ in degrees of in the change in gene expression profile and the change in DNA methylation pattern in association with reprogramming, the acquisition rate and production efficiency may vary significantly even in a case where, by using the same protocol, they are subsequently induced to differentiate into a specific cell such as a myocardial cell. At present, it is difficult to make the differences present between these clones uniform, and it is customary to select and culture a clone most suitable for production. In addition, even in a case where the same clone is used for production, the acquisition rate and productivity often differ for each production and batch due to the influences of the incubator's technology and protocol robustness, and thus it is not easy to analyze the factors thereof and take countermeasures.

Regarding the production period of a cell product, it takes several weeks to one month to obtain a specific cell such as a myocardial cell by carrying out a differentiation induction treatment on a pluripotent stem cell such as an iPS cell. Moreover, culture media that are used in cell culture and drugs that are used in the differentiation induction treatment are expensive. However, in a case where it is possible to sort a pluripotent stem cell that is expected to have a high acquisition rate or to predict a differentiation induction result, at a relatively early stage in the production process of the cell product, it is possible to take measures regarding pluripotent stem cells that cannot be expected to have a high acquisition rate, for example, discontinuing treatments or changing production conditions, whereby it is possible to increase the productivity of the cell product and reduce the production cost.

The disclosed technology has been made in consideration of the above-described points, and an object of the disclosed technology is to provide a sorting method for a pluripotent stem cell, which can contribute to the improvement of productivity of a cell product, a prediction method for a differentiation induction result, and a production method for a cell product.

A sorting method for a pluripotent stem cell according to the disclosed technology comprises generating a phase contrast image of an aggregate of a pluripotent stem cell from a hologram in which the aggregate is captured; deriving a phase contrast amount density obtained by dividing a total phase contrast amount which is a value obtained by integrating a phase contrast amount of each of a plurality of pixels that constitute the phase contrast image, by a volume of the aggregate; and sorting the pluripotent stem cell based on the phase contrast amount density.

The sorting of pluripotent stem cells may be sorting a clone containing an aggregate having the phase contrast amount density which is within a predetermined range, sorting a production batch containing an aggregate having the phase contrast amount density which is within a predetermined range, sorting a plate containing an aggregate having the phase contrast amount density which is within a predetermined range, sorting an aggregate having the phase contrast amount density which is within a predetermined range, or sorting pluripotent stem cells contained in an aggregate having the phase contrast amount density which is within a predetermined range.

Further, such a pluripotent stem cell sorted as described above may be targeted for a differentiation induction treatment for differentiation into a specific cell.

The above-described predetermined range of the phase contrast amount density may be determined based on a relationship between the phase contrast amount density acquired in regard to the aggregate before carrying out the differentiation induction treatment and an acquisition rate indicating a proportion of the number of the specific cells obtained by the differentiation induction treatment with respect to the number of pluripotent stem cells at a time before carrying out the differentiation induction treatment.

A prediction method for a differentiation induction result according to the disclosed technology comprises generating a phase contrast image of an aggregate of a pluripotent stem cell from a hologram in which the aggregate is captured; deriving a phase contrast amount density obtained by dividing a total phase contrast amount which is a value obtained by integrating a phase contrast amount of each of a plurality of pixels that constitute the phase contrast image, by a volume of the aggregate; and deriving, based on the phase contrast amount density, a predicted value regarding the number of specific cells obtained by carrying out a differentiation induction treatment for differentiating the pluripotent stem cell into the specific cell.

For example, the predicted value may be an acquisition rate indicating a proportion of the number of the specific cells obtained by the differentiation induction treatment with respect to the number of pluripotent stem cells at a time before carrying out the differentiation induction treatment.

A function that shows a relationship between the phase contrast amount density and the acquisition rate may be used to derive the predicted value.

A production method for a cell product according to the disclosed technology comprises a culture step of culturing a pluripotent stem cell; a sorting step of sorting the pluripotent stem cell cultured in the culture step; and a differentiation induction step of carrying out a differentiation induction treatment for differentiating the pluripotent stem cell sorted in the sorting step into a specific cell. The sorting step includes generating a phase contrast image of an aggregate of a pluripotent stem cell from a hologram in which the aggregate is captured; deriving a phase contrast amount density obtained by dividing a total phase contrast amount which is a value obtained by integrating a phase contrast amount of each of a plurality of pixels that constitute the phase contrast image, by a volume of the aggregate; and sorting the pluripotent stem cell based on the phase contrast amount density.

In each of the methods according to the disclosed technology, the specific cell may be a myocardial cell.

In each of the methods according to the disclosed technology, the phase contrast amount density is preferably derived based on the phase contrast image generated from the hologram in which the aggregate before carrying out the differentiation induction treatment is captured.

According to the disclosed technology, there is provided a sorting method for a pluripotent stem cell, which can contribute to the improvement of productivity of a cell product, a prediction method for a differentiation induction result, and a production method for a cell product.

BRIEF DESCRIPTION OF THE DRAWINGS

Fig. 1 is a step flow chart showing one example of a production method for a cell product.
Fig. 2 is a flow chart showing one example of a processing flow in a sorting step according to an embodiment of the disclosed technology.
Fig. 3 is a view illustrating one example of an image capturing system according to the embodiment of the disclosed technology.

Fig. 4A is an image showing one example of a hologram of a plurality of spheres according to the embodiment of the disclosed technology.

Fig. 4B is an image showing one example of a Fourier transformed image of a plurality of spheres according to the embodiment of the disclosed technology.

Fig. 4C is an image showing one example of a phase contrast image of a plurality of spheres before unwrapping according to the embodiment of the disclosed technology.

Fig. 4D is an image showing one example of a phase contrast image of a plurality of spheres after unwrapping according to the embodiment of the disclosed technology.

Fig. 5 is a view illustrating a concept of a phase contrast image according to the embodiment of the disclosed technology.

In Fig. 6, a graph on the left is a graph illustrating one example of a relationship between the position in the plane direction and the phase contrast amount in a phase contrast image of spheres, and a graph on the right is a histogram of the phase contrast amount in the phase contrast image of the sphere.

Fig. 7 is a graph showing one example of a relationship between the focal position and the variation in the phase contrast amount in phase contrast images of spheres.

Fig. 8 is a graph showing one example of a relationship between the phase contrast amount density of the sphere of iPS cells and the acquisition rate of myocardial cells obtained by the differentiation induction treatment on the iPS cells.

Fig. 9 is a graph showing a relationship between a predicted value of an acquisition rate of myocardial cells and a measured value thereof.

DESCRIPTION OF THE PREFERRED EMBODIMENTS

Hereinafter, embodiments of the disclosed technology will be described with reference to the drawings. It is noted that in each of the drawings, substantially the same or equivalent constitution elements or parts are designated by the same reference numeral.

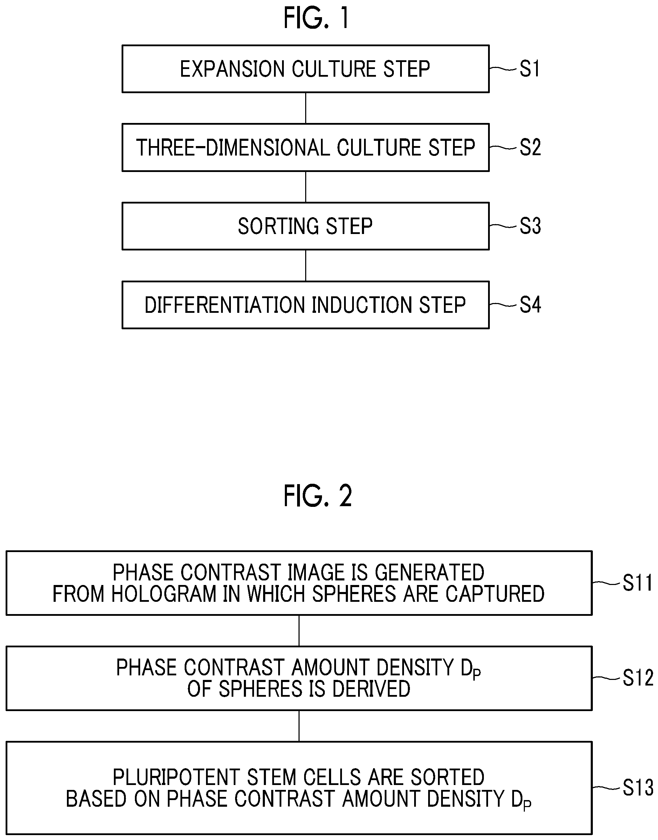

Fig. 1 is a step flow chart illustrating one example of a production method for a cell product according to the embodiment of the disclosed technology. The production method for a cell product according to the present embodiment is a production method for obtaining a specific cell that has deviated from the undifferentiated state, such as a myocardial cell, by differentiation induction of a pluripotent stem cell such as an iPS cell. The production method for a cell product according to the present embodiment includes an expansion culture step S1, a three-dimensional culture step S2, a sorting step S3, and a differentiation induction step S4.

In the expansion culture step S1, the expansion culture of proliferating a pluripotent stem cell is carried out. The expansion culture is carried out, for example, by two-dimensional culture in which a plurality of pluripotent stem cells are adhered on a base material. As the base material, it is possible to use, for example, a commercially available multi-well plate for cell culture. In the expansion culture step S1, culture medium exchange and subculture treatment are carried out a plurality of times until the desired number of pluripotent stem cells are obtained. It is also possible to carry out expansion culture by the three-dimensional culture in which pluripotent stem cells are cultured in a state of being suspended in a culture medium.

In the three-dimensional culture step S2, the pluripotent stem cell is cultured to form a spherical aggregate (hereinafter, referred to as a sphere). During the transition from the two-dimensional culture to the three-dimensional culture, pluripotent stem cells adhered on the base material are detached from the base material using an enzyme agent such as trypsin. The detached pluripotent stem cells are subjected to, for example, spinner culture in a container containing a culture medium. The pluripotent stem cells form spherical spheres while being proliferated in a container. It is noted that in this step, it is also possible to apply stationary culture in which pluripotent stem cells are cultured in a stationary state in a culture medium. In a case of stationary culture, a plate subjected to a non-cell adhesive surface treatment may be used, or a material for preventing sphere sediment may be added to the culture medium. Examples of the sedimentation prevention material include heteropolysaccharides polymers such as Gellan Gum.

In the sorting step S3, a pluripotent stem cell is sorted. That is, in this step, a pluripotent stem cell to be targeted for a differentiation induction treatment that is carried out in the differentiation induction step S4 is specified. Details of the sorting method for a pluripotent stem cell will be described later.

In the differentiation induction step S4, the pluripotent stem cells targeted for a differentiation induction treatment in the sorting step S3 are subjected to a differentiation induction treatment for differentiation into specific cells. The differentiation induction treatment is carried out at the timing when a predetermined period (for example, 2 days) has elapsed from the start of the three-dimensional culture step S2. For example, in a case where myocardial cells are obtained from pluripotent stem cells, a physiologically active substance that induces differentiation into mesoderm is added to the culture medium. Specific examples of the physiologically active substance include bFGF, Activin, BMP4.

Then, a Wnt signal inhibitor that induces differentiation into myocardial cells is added to the culture medium. Specific examples of the Wnt signal inhibitor include XAV939.

Fig. 2 is a flow chart showing a processing flow that is carried out in the sorting step S3. In the step S11, a phase contrast image of spheres is generated from the hologram obtained by capturing an image of the spheres formed in the three-dimensional culture step S2. Holography is carried out, for example, on the spheres two days after the start of the three-dimensional culture step S2. Details of the holography and the phase contrast image will be described later.

In a step S12, the phase contrast amount density $D_p$ of the sphere is derived based on the phase contrast image generated in the step S11. The phase contrast amount density Dp is determined by dividing the total phase contrast amount $P_A$ which is a value obtained by integrating the phase contrast amount of each of a plurality of pixels that constitute the phase contrast image, by the volume of the sphere. The details of the total phase contrast amount $P_A$ and the phase contrast amount density Dp will be described later.

In a step S13, pluripotent stem cells are sorted based on the phase contrast amount density $D_P$ derived in the step S12. Specifically, in a case where the phase contrast amount density $D_P$ is within a predetermined range, the pluripotent stem cells contained in the sphere are targeted for a differentiation induction treatment. On the other hand, in a case where the phase contrast amount density Dp is not within the above-described predetermined range, the pluripotent stem cells contained in the sphere are excluded from targets for the differentiation induction treatment.

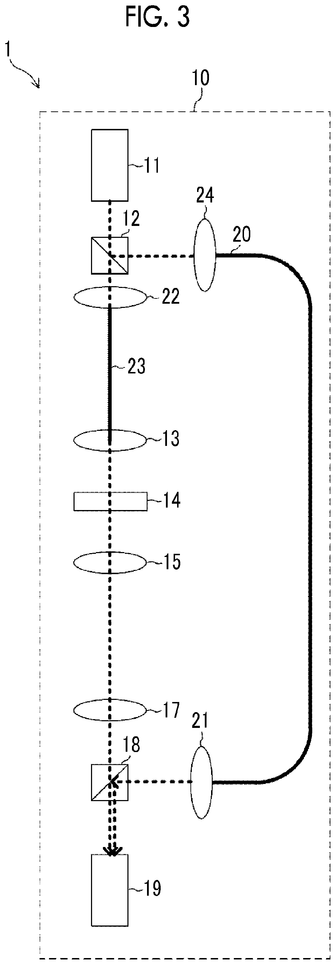

Fig. 3 is a view illustrating one example of a constitution of an image capturing system 1 that is used for sorting a pluripotent stem cell in the sorting step S3. The image capturing system 1 is constituted by including a hologram optical system 10 for acquiring a hologram of spheres using a known digital holography technology,

The digital holography technology is a technology that restores wavefronts of light waves from an object by capturing, with an image sensor, an image generated due to the interference between the object light that has penetrated through or reflected from the object and the reference light that is coherent to the object light and subjecting an image obtained by the image capturing to a numerical calculation based on light propagation. According to the digital holography technology, it is possible to quantify the phase distribution of an object and acquire three-dimensional information of the object without mechanically moving the focal position.

A hologram optical system 10 includes a laser beam source 11, beam splitters 12 and 18, collimating lens 13, 21, 22, and 24, an objective lens 15, an imaging lens 17, and a complementary metal oxide semiconductor (CMOS) camera 19. A sphere as a sample 14 set on a sample stage is arranged between the collimating lens 13 and the objective lens 15.

As the laser beam source 11, it is possible to use, for example, a HeNe laser having a wavelength of 632.8 nm. The laser beams emitted from the laser beam source 11 are split into two laser beams by the beam splitter 12. One of the two laser beams serves as object light and the other thereof serves as reference light. The object light is incident on an optical fiber 23 by the collimating lens 22 and guided to the front of the collimating lens 13 by the optical fiber 23. The object light is made to be parallel light by the collimating lens 13 and then emitted on the sphere which is the sample 14 set on the sample stage. As the optical fiber 23, it is possible to use, for example, an optical fiber having NA = 0.11. As the collimating lens 13, it is possible to use, for example, a collimating lens having f = 10 mm (NA = 0.4). The diameter of the laser beam that is emitted to the sphere is, for example, 2.2 mm. The image generated due to the object light penetrated through the sphere is magnified by the objective lens 15. The object light penetrated through the objective lens 15 is made to be parallel light again by the imaging lens 17 and then an image is formed on the imaging surface of the CMOS camera 19 through the beam splitter 18. As the objective lens 15, it is possible to use, for example, an object lens having NA = 0.45 and f = 20 mm. As the imaging lens 17, it is possible to use, for example, an object lens having f = 200 mm and an opening diameter of 36 nm, and an image is formed on the imaging surface of the CMOS camera at an image magnification of 10 times. On the other hand, the reference light is incident on an optical fiber 20 by the collimating lens 24 and guided to the front of the collimating lens 21 by the optical fiber 20. The reference light emitted from the optical fiber 20 is made to be parallel light by the collimating lens 21 and is incident on the imaging surface of the CMOS camera 19 through the beam splitter 18. As the collimating lens 21, it is possible to use, for example, a collimating lens having f = 100 mm (NA = 0.25). The diameter of the laser beam that is emitted from the collimating lens 21 is, for example, 22 mm. A hologram generated due to the interference between the object light and the reference light is recorded by the CMOS camera 19. As the CMOS camera 19, it is possible to use, for example, a monochrome image sensor having a resolution of 2,448 × 2,048 and a sensor size of 3.45 μm × 3.45 μm. Image capturing may be carried out by tilting the beam splitter 18 so that the reference light is tilted by about 3° with respect to the object light, so that an off-axial optical system in which optical axis directions of the object light and the reference light, incident on the imaging surface of the CMOS camera 19, are different from each other.

According to the image capturing system 1 according to the present embodiment, it is possible to acquire a phase contrast image of a sphere without destroying the sphere and without damaging cells that constitute the sphere. It is noted that the constitution of the image capturing system 1 described above is merely one example and thus is not limited to the constitution described above. For carrying out the sorting method according to the present embodiment, it is possible to use any image capturing system capable of acquiring a hologram by using digital hologram technology.

Hereinafter, a description will be made for one example of a method of acquiring a phase contrast image of a sphere from a hologram of spheres acquired by using the image capturing system 1.

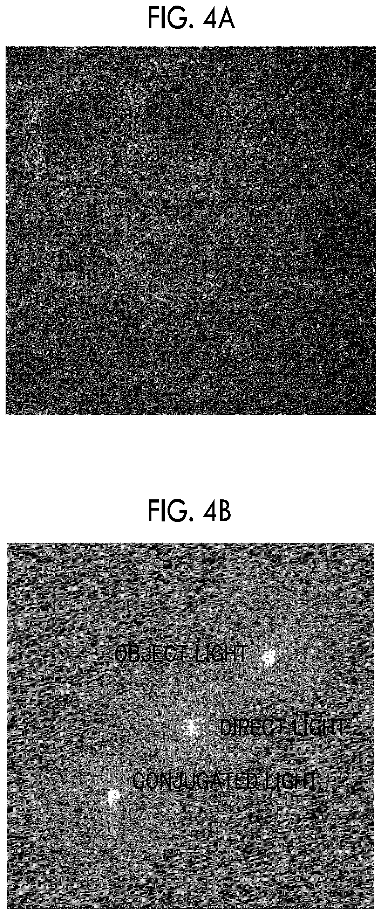

First, a hologram of spheres, acquired by the image capturing system 1 and exemplified in Fig. 4A, is trimmed to a size of, for example, 2,048 × 2,048 and then subjected to a two-dimensional Fourier transform. Fig. 4B is one example of a Fourier transformed image of the spheres obtained by this processing. Fig. 4B shows an image based on direct light, object light, and conjugated light.

Next, the position of the object light is specified by specifying the amount of deviation of the object light with respect to the direct light in the Fourier transformed image, and the complex amplitude component of only the object light is extracted by, for example, the frequency filtering process using a mask having a circular opening of a radius of 250 pixels.

Next, for example, the angular spectral method is applied to restore an image showing the phase of the sphere at any spatial position. Specifically, the angular spectrum U ($f_x$, $f_y$; 0) of the Fourier transformed image of the wavefront u (x, y; 0) captured on the imaging surface of the CMOS camera 19 is determined. Next, as shown in Expression (1) below, a transfer function H ($f_x$, $f_y$; z) is multiplied by the angular spectrum U ($f_x$, $f_y$; 0) to reproduce the wavefront at any position z in the optical axis direction (the z direction) of the image capturing system 1. Here, the transfer function H ($f_x$, $f_y$; z) is a frequency response function (a Fourier transform of the impulse response function (the Green's function)).

$$U\left(f_x, f_y; z\right) = U\left(f_x, f_y; 0\right) H\left(f_x, f_y; z\right), \quad H = e^{\pm \frac{2\pi}{\lambda}\sqrt{1-(\lambda f_x)^2-(\lambda f_y)^2}} \quad \cdots (1)$$

Next, as shown in Expression (2), the wavefront U ($f_x$, $f_y$; z) at the position z in the optical axis direction (the z direction) of the image capturing system 1 is subjected to the inverse Fourier transform, whereby a solution u (x, y; z) at the position z is derived.

$$\begin{aligned} u(x, y; z) &= F^{-1}\left[U\left(f_x, f_y; z\right)\right] \\ &= F^{-1}\left[U\left(f_x, f_y; 0\right) H\left(f_x, f_y; z\right)\right] \\ &= F^{-1}\left[F\left[u(x, y; 0)\right] H\left(f_x, f_y; z\right)\right] \end{aligned} \quad \cdots (2)$$

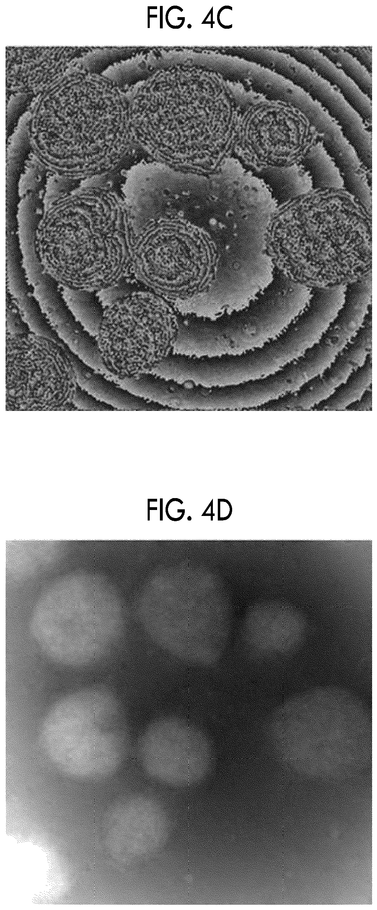

Next, as shown in Expression (3), a phase contrast image is generated by deriving the phase φ for u (x, y; z). Fig. 4C is one example of a phase contrast image of the spheres before unwrapping, obtained by each processing described above.

$$\phi = \arctan\left(\frac{\operatorname{Im}(u)}{\operatorname{Re}(u)}\right) \quad \cdots (3)$$

The phase of the sphere before unwrapping shown in Fig. 4C is convoluted to a value of 0 to $2\pi$. Here, in a case where a part of $2\pi$ or more is joined by applying a phase connection (unwrapping) method, for example, the unweighted least squares method or the Flynn's algorithm, it is possible to obtain a final phase contrast image of the spheres as shown in Fig. 4D. It is noted that a large number of unwrapping methods have been proposed, and a suitable method that does not cause phase mismatch may be appropriately selected.

In a case where each processing described above is carried out, it is possible to generate a phase contrast image at each of the positions z different from each other in the optical axis direction (the z direction) of the image capturing system 1.

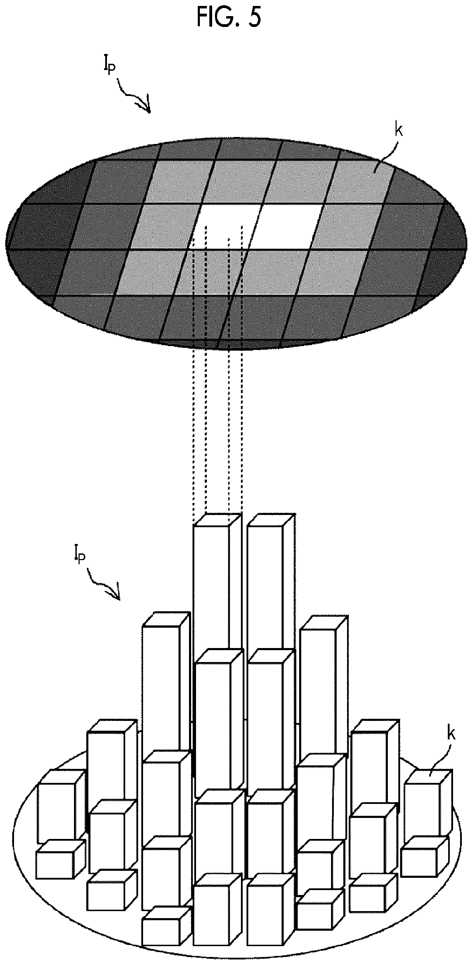

Hereinafter, the phase contrast image will be described. Fig. 5 is a view illustrating a concept of a phase contrast image Ip. The lower part of Fig. 5 is a view in which a phase contrast amount in each pixel k of the phase contrast image Ip is three-dimensionally displayed. The upper part of Fig. 5 is a view in which the phase contrast amount in each pixel k of the phase contrast image Ip is illustrated on a plane in gray scale.

Here, in the same focal plane of the phase contrast image Ip, in a case where the phase of the background (the region where spheres are not present) present is denoted by $P_B$, and the phase of the region where spheres are present is denoted by Ps, a phase contrast amount P in the phase contrast image Ip is expressed by Expression (4). It is noted that the term "phase" in the present specification is the phase of the electric field amplitude in a case where light is regarded as an electromagnetic wave and is used in a more general meaning.

$$P = P_S - P_B \quad \cdot \cdot \cdot \cdot (4)$$

Further, a phase contrast amount $P_k$ in each pixel k of the phase contrast image Ip can be expressed by Expression (5). Here, $n_k$ is the refractive index of the sphere at the portion corresponding to each pixel k of the phase contrast image $I_P$, $d_k$ is the thickness of the sphere at the portion corresponding to each pixel k of the phase contrast image Ip, and $\lambda$ is the wavelength of the object light in the hologram optical system 10.

$$P_k = 2\pi \frac{n_k \cdot d_k}{\lambda} \quad \cdot \cdot \cdot \cdot (5)$$

The phase contrast image of the sphere is an image showing the optical path length distribution of the object light penetrated through the sphere. Since the optical path length in the sphere corresponds to the product of the refractive index of the sphere and the thickness of the sphere, the phase contrast image of the sphere contains information on the refractive index and the thickness (the shape) of the sphere, as also shown in Expression (5).

From the phase contrast image that is out of focus with respect to the sphere, it is not possible to obtain accurate information that matches the actual condition of the sphere due to the influence of the spread caused by diffraction. As a result, it is preferable to focus on the sphere in a case of acquiring a phase contrast image from the hologram acquired by the CMOS camera 19. Here, "to focus on the sphere" means to obtain a phase contrast image that is sliced near the center of the spherical sphere. In a case where the state of the sphere is determined using a phase contrast image in which the sphere is in focus, it is possible to obtain a more accurate determination result.

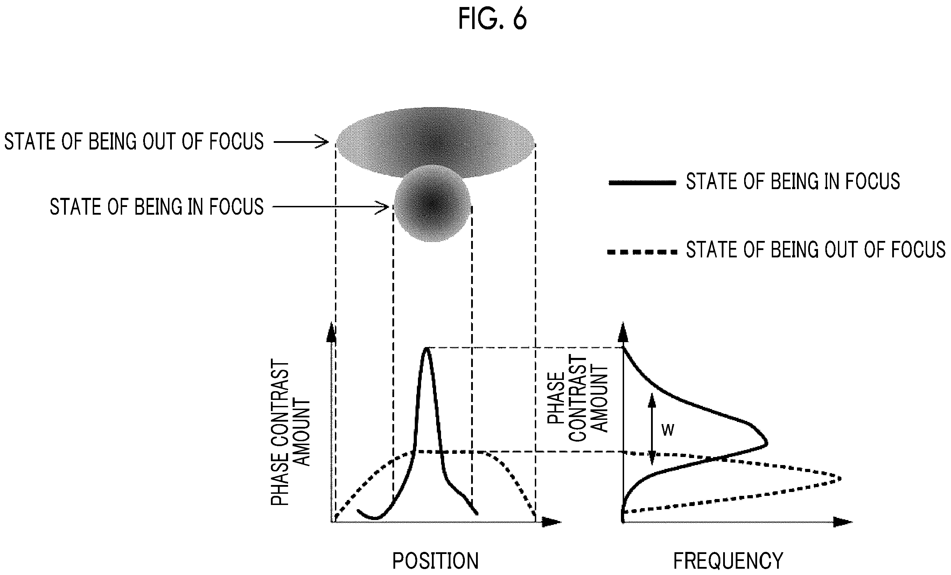

The graph on the left of Fig. 6 is a graph showing one example of the relationship between the position in the plane direction and the phase contrast amount of the sphere in the phase contrast image, where the solid line corresponds to a state where the sphere is in focus and the dotted line corresponds to a state where the sphere is out of focus. In a case where the sphere is in focus, a steep peak appears at a specific position in the phase contrast image. On the other hand, in a case where the sphere is out of focus, a peak is low and gentle as compared with the case of being in focus.

The graph on the right of Fig.6 is a histogram of the phase contrast amount in the phase contrast image of the sphere, where the solid line corresponds to a state where the sphere is in focus and the dotted line corresponds to a state where the sphere is out of focus. In a case where the sphere is in focus, the curve width w (the variation in the phase contrast amount) is relatively large, and in a case where the sphere is out of focus, the curve width w (the variation in the phase contrast amount) is relatively small.

As a result, focusing can be achieved by acquiring the phase contrast image of the sphere for each of the focal positions (slice positions) different from each other, determining the curve width w (the variation in the phase contrast amount) in the histogram of the phase contrast amount for each acquired phase contrast image, and extracting a phase contrast image having the maximum width w among the determined widths w as the phase contrast image in which the sphere is in focus.

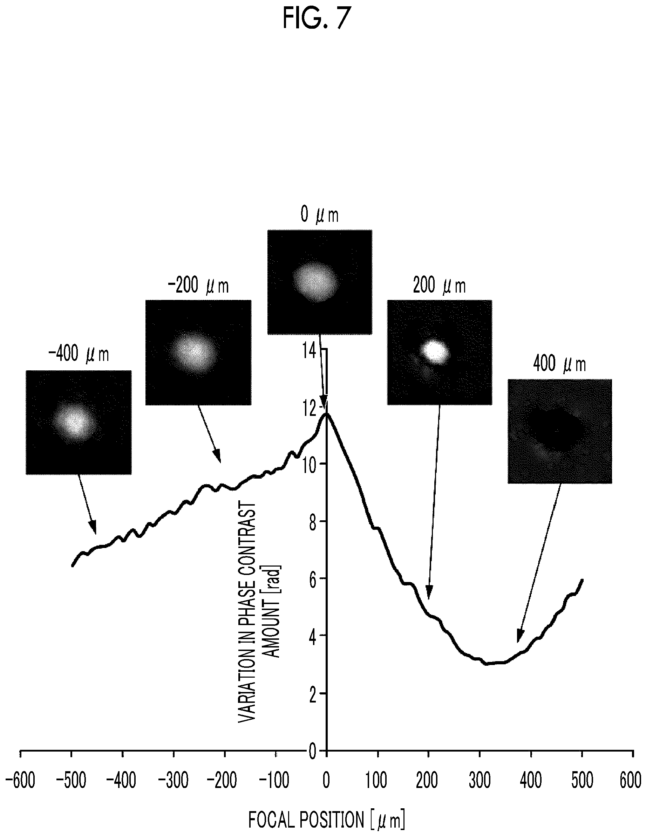

Fig. 7 is a graph showing one example of a relationship between the focal position (the slice position) and the variation in the phase contrast amount in phase contrast images of spheres. In Fig. 7, phase contrast images of spheres corresponding to focal positions of -400 $\mu$m, -200 $\mu$m, 0 $\mu$m, +200 $\mu$m, and +400 $\mu$m are exemplified together with the graph. In Fig. 7, the focal position where the variation in the phase contrast amount has the maximum value is set to 0 $\mu$m. According to the above-described autofocus processing, a phase contrast image corresponding to a focal position of 0 $\mu$m where the variation in the phase contrast amount has the maximum value is extracted as the in-focus phase contrast image. In the phase contrast image corresponding to a focal position of 0 $\mu$m where the variation in the phase contrast amount has the maximum value, the contour of the sphere becomes clearest.

The above-described total phase contrast amount $P_A$ is expressed by Expression (6). Here, s is the area of each pixel k of the phase contrast image, and $v_k$ is the volume of the sphere at the portion corresponding to each pixel k of the phase contrast image. As shown in Expression (6), the total phase contrast amount $P_A$ corresponds to the summation obtained by integrating the phase contrast amount $P_k$ for every pixel of the phase contrast image of the sphere for all the pixels k. The pixel value of the phase contrast image corresponds to the phase contrast amount $P_k$.

$$P_A = \sum_{k=0}^{N} P_k \cdot s = \frac{2\pi}{\lambda} \sum_{k=0}^{N} n_k \cdot d_k \cdot s = \frac{2\pi}{\lambda} \sum_{k=0}^{N} n_k \cdot v_k \quad \cdots (6)$$

The phase contrast amount density $D_P$ is expressed by Expression (7). Here, V is the volume of the sphere. As shown in Expression (7), the phase contrast amount density Dp corresponds to a total phase contrast amount $P_A$ divided by the volume V of the sphere. It is conceived that live cells maintain a constant value of an internal refractive index, which is different from the refractive index of the medium, due to the homeostasis thereof. On the other hand, it is conceived that dead cells lose homeostasis and thus the internal refractive index becomes substantially the same as the refractive index of the medium. As a result, the phase contrast amount density Dp can be used as an indicator indicating the state of cells. It is noted that $2\pi/\lambda$ can be treated as a constant, and thus the multiplication of $2\pi/\lambda$ may be omitted in a case of deriving the phase contrast amount density Dp. Here, in a case where the volume average refractive index difference Nave of the sphere is expressed as Nave = $\Sigma n_k(v_k/V)$, Expression (7) is expressed as $D_P = (2\pi/\lambda) \times N_{ave}$, and thus the phase contrast amount density is a phase contrast amount density obtained by normalizing the volume-averaged difference in the refractive index of the spheres with the length of the wavelength. The volume V of the sphere can be determined by calculating the sphere equivalent diameter from the cross-sectional image of the phase image of the sphere. More accurately, it is also possible to calculate the ellipsoidal sphere equivalent diameter. It is noted that in the disclosed technology, the volume average refractive index difference $N_{ave}$ can be treated as an indicator equivalent to the phase contrast amount density Dp.

$$D_P = \frac{P_A}{V} = \frac{2\pi}{\lambda} \sum_{k=0}^{N} n_k \cdot \frac{v_k}{V} \quad \cdots (7)$$

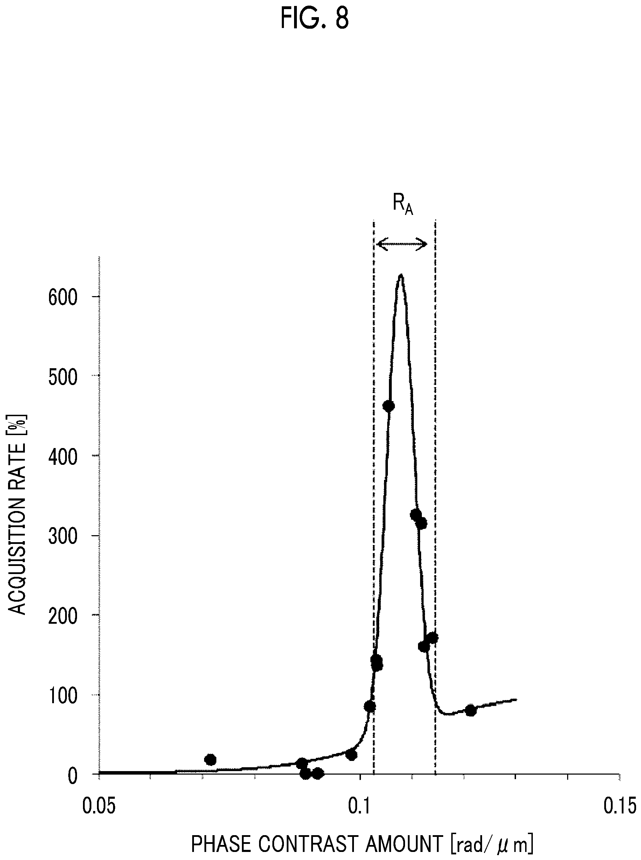

Fig. 8 is a graph showing one example of a relationship between the phase contrast amount density $D_p$ of spheres of iPS cells and the acquisition rate Y of myocardial cells obtained by the differentiation induction treatment on the iPS cells. The phase contrast amount density $D_P$ is a phase contrast amount density derived from a phase contrast image generated from the hologram acquired in regard to the sphere at a time of carrying out the differentiation induction treatment (in the present embodiment, two days after the start of the three-dimensional culture step S2). Each plot shown in Fig. 8 corresponds to each of a plurality of clones in the production process of the cell product, and the phase contrast amount density $D_p$ exhibited by each plot is the average value of the phase contrast amount densities $D_p$ acquired in regard to a plurality of spheres in the production lot.

The acquisition rate Y is expressed by Expression (8). In Expression (8), $N_i$ is the number of pluripotent stem cells in the production lot at a time before carrying out the differentiation induction treatment (in the present embodiment, the first day of the start of the three-dimensional culture step S2), and $N_c$ is the number of myocardial cells in the production lot obtained at a time after carrying out the differentiation induction treatment (12 days after the start of the differentiation induction step S4 in the present embodiment). That is, the acquisition rate Y is the proportion of the number $N_c$ of the produced myocardial cells to the number $N_i$ of the pluripotent stem cells introduced. It is noted that cells proliferate even between at a time of measuring $N_i$ and at a time of measuring $N_c$, and thus the acquisition rate Y may exceed 100%. In addition, the number of myocardial cells $N_c$ can be acquired by measuring the number of cTnT positive cells.

$$Y = \frac{N_c}{N_i} \times 100 \quad \cdots (8)$$

As shown in Fig. 8, in pluripotent stem cells contained in a sphere having a phase contrast amount density Dp within the predetermined range $R_A$, the acquisition rate Y of myocardial cells obtained by a subsequent differentiation induction treatment becomes significantly higher. That is, Fig. 8 shows that pluripotent stem cells in which a high acquisition rate Y is obtained can be sorted with the phase contrast amount density Dp of the sphere.

The sorting method for a pluripotent stem cell according to the embodiment of the disclosed technology, illustrated in Fig. 2, is a sorting method based on the finding that the acquisition rate Y of myocardial cells is reflected in the phase contrast amount density $D_p$ of the sphere, as shown in Fig. 8, and it includes sorting the pluripotent stem cells based on the phase contrast amount density $D_p$ (see a step S13 in Fig. 2). Specifically, it includes targeting pluripotent stem cells contained in a sphere in which the phase contrast amount density $D_p$ is within the predetermined range $R_A$, for a differentiation induction treatment; and excluding pluripotent stem cells contained in a sphere in which the phase contrast amount density $D_p$ is not within the predetermined $R_A$ from targets for the differentiation induction treatment.

The sorting method according to the embodiment of the disclosed technology includes determining the predetermined range $R_A$ for the sorting in regard to the phase contrast amount density $D_p$, based on the relationship between the phase contrast amount density $D_p$ acquired in regard to the sphere before carrying out the differentiation induction treatment and the acquisition rate Y of myocardial cells obtained by the differentiation induction treatment on the pluripotent stem cells contained in this sphere, as exemplified in Fig. 8.

For example, in the example shown in Fig. 8, in a case where the acquisition rate of myocardial cells is made to be 100% or more, pluripotent stem cells contained in the sphere of which the phase contrast amount density Dp is within a range of 0.102 [rad/$\mu$m] or more and 0.114 [rad/$\mu$m] or less are targeted for a differentiation induction treatment. It is noted that since the phase contrast amount density Dp changes depending on the wavelength $\lambda$ of the object light in the hologram optical system 10, the wavelength $\lambda$ is preferably always constant.

According to the sorting method according to the embodiment of the disclosed technology, since pluripotent stem cells are sorted based on the phase contrast amount density $D_p$ of spheres, it is possible to specify a pluripotent stem cell that can be expected to have a desired acquisition rate at a relatively early stage in the production process of the cell product. As a result, it is possible to take measures regarding pluripotent stem cells that cannot be expected to have a desired acquisition rate, for example, discontinuing treatments or changing production conditions, whereby it is possible to increase the productivity of the cell product and reduce the production cost.

In the sorting method according to the embodiment of the disclosed technology, the phase contrast amount density $D_p$ is preferably derived based on the phase contrast image generated from the hologram in which the sphere before carrying out the differentiation induction treatment is captured. As a result, pluripotent stem cells can be sorted at a stage before carrying out the differentiation induction treatment, and thus the effect of increasing the productivity of a cell product can be promoted. The measurement of the phase contrast amount density Dp is preferably carried out after 2 hours and within 5 days, more preferably carried out after 12 hours and within 3 days, and most preferably carried out after 1 day and within 2 days from the start of the spinner culture. In a case where stationary culture is carried out instead of spinner culture to form spheres, the measurement of the phase contrast amount density Dp is preferably carried out after 2 hours and within 5 days, more preferably carried out after 12 hours and within 3 days, and most preferably carried out after 1 day and within 2 days from the start of stationary culture for forming spheres. Even after the differentiation induction treatment, the relationship between the phase contrast amount density Dp and the acquisition rate Y is maintained in a period until the change in phase contrast amount density Dp associated with differentiation appears, and thus the phase contrast amount density Dp may be derived based on the phase contrast image generated from the hologram in which spheres have been captured within this period.

The sorting of pluripotent stem cells can be carried out on a sphere basis or on a production lot basis. In a case where the sorting of pluripotent stem cells is carried out on a production lot basis, for example, the phase contrast amount density Dp is acquired in regard to a part or all of a plurality of spheres in the production lot, and then all pluripotent stem cells contained in this production lot may be targeted for a differentiation induction treatment in a case where the intra-lot average value of the phase contrast amount density Dp is within a predetermined range.

Here, the relationship between the phase contrast amount density $D_p$ and the acquisition rate Y can be mathematically expressed using a known function fitting method. For example, using a linear combination model of the Gaussian function and the sigmoid function, it is possible to derive Expression (9) as a function that shows the relationship between the phase contrast amount density Dp and the acquisition rate Y, shown in Fig. 8.

$$Y = \frac{C}{B\sqrt{2\pi}} e^{\frac{(D_P - A)^2}{2B^2}} + \frac{D}{1 + e^{-E(D_P - A)}} + F \quad \cdots (9)$$

A = $1.078588 \times 10^{-1}$

B = $2.638946 \times 10^{-3}$

C = 3.0805526

D=1.010000 × 10²

E=1.010000 × 10²

F=1.077393

Expression (9) shows that it is possible to predict the acquisition rate Y of myocardial cells that are obtained by the differentiation induction treatment from the phase contrast amount density $D_P$ of spheres that are obtained in the middle stage in the production process of a cell product. The prediction method for a differentiation induction result according to the embodiment of the disclosed technology is a prediction method that utilizes the relationship between the phase contrast amount density Dp and the acquisition rate Y of myocardial cells, and it includes, based on the phase contrast amount density $D_p$ of the sphere, deriving a predicted value regarding the number of myocardial cells obtained by carrying out a differentiation induction treatment on a plurality of pluripotent stem cells contained in this sphere. Specifically, it includes substituting the phase contrast amount density $D_p$ of the sphere in a function that shows a relationship between the phase contrast amount density $D_p$ and the acquisition rate Y, the function being exemplified in Expression (9), thereby deriving a predicted value of the acquisition rate Y of myocardial cells that are obtained by subjecting pluripotent stem cells contained in this sphere have been to the differentiation induction treatment.

In this manner, in a case where a function that shows a relationship between the phase contrast amount density Dp and the acquisition rate Y is specified, it is possible to derive, based on the phase contrast amount density Dp of the sphere, a predicted value of the acquisition rate Y of myocardial cells that are obtained in a case where the differentiation induction treatment has been carried out on this sphere, that is, it is possible to predict the differentiation induction result. In a function that shows a relationship between the phase contrast amount density Dp and the acquisition rate Y, it is also possible to derive a predicted value other than the acquisition rate as a predicted value regarding the number of myocardial cells. For example, in a case where $N_i$ (the number of pluripotent stem cells introduced) in Expression (8) has been measured, it is also possible to derive a predicted value of the number of myocardial cells $N_c$ (= $N_i$ × E) to be produced, by using the function that shows a relationship between the phase contrast amount density $D_p$ and the acquisition rate Y of myocardial cells.

As described above, according to the prediction method for the differentiation induction result according to the embodiment of the disclosed technology, a predicted value regarding the number of myocardial cells obtained by carrying out a differentiation induction treatment on pluripotent stem cells contained in the sphere is derived based on the phase contrast amount density $D_p$ of this sphere. As a result, it is possible to take measures, for example, regarding pluripotent stem cells in which the derived predicted value is less than the required level, for example, discontinuing treatments or changing production conditions, whereby it is possible to increase the productivity of the cell product and reduce the production cost.

In the prediction method for a differentiation induction result according to the embodiment of the disclosed technology, the phase contrast amount density $D_p$ is preferably derived based on the phase contrast image generated from the hologram in which the sphere before carrying out the differentiation induction treatment is captured. As a result, the differentiation induction result can be predicted at a stage before carrying out the differentiation induction treatment, and thus the effect of increasing the productivity of a cell product can be promoted. The measurement of the phase contrast amount density Dp is preferably carried out after 2 hours and within 5 days, more preferably carried out after 12 hours and within 3 days, and most preferably carried out after 1 day and within 2 days from the start of the spinner culture. In a case where stationary culture is carried out instead of spinner culture to form spheres, The measurement of the phase contrast amount density Dp is preferably carried out after 2 hours and within 5 days, more preferably carried out after 12 hours and within 3 days, and most preferably carried out after 1 day and within 2 days from the start of stationary culture for forming spheres. Even after the differentiation induction treatment, the relationship between the phase contrast amount density Dp and the acquisition rate Y is maintained in a period until the change in phase contrast amount density Dp associated with differentiation appears, and thus the phase contrast amount density Dp may be derived based on the phase contrast image generated from the hologram in which spheres have been captured within this period.

In the above description, a case where pluripotent stem cells are differentiated into myocardial cells has been exemplified; however, the disclosed technology is not limited to this aspect. It is conceived that the relationship between the phase contrast amount density $D_p$ and the acquisition rate Y, exemplified in Fig. 8, is also established in a case where pluripotent stem cells are differentiated into cells other than myocardial cells. Accordingly, it is conceived that the disclosed technology can be applied even in a case where pluripotent stem cells are differentiated into cells other than myocardial cells. Examples of the cell other than the myocardial cell include entodermal cells such as digestive system cell (a hepatocyte, cholangiocyte, a pancreatic endocrine cell, an acinar cell, a ductal cell, an absorptive cell, a goblet cell, a Paneth cell, an intestinal endocrine cells, and the like), and cells of tissues such as lung and thyroid, examples of the mesenchymal cell include blood cells and lymphoid cells (a hematopoietic stem cell, an erythrocyte, a platelet, a macrophage, a granulocyte, a helper T cell, a killer T cell, a B lymphocyte, and the like), vascular system cells (a vascular endothelial cell and the like), an osteoblast, a bone cell, a cartilage cell, a tendon cell, an adipocyte, a skeletal muscle cell, and a smooth muscle cell, and examples of the ectodermal cell include a neural cell, sensory organ (crystalline lens, retina, inner ear, and the like) cells, a skin epidermal cell, a hair follicle.

[Example]

Hereinafter, Example relating to the derivation of a graph showing a relationship between the phase contrast amount density Dp and the acquisition rate Y, shown in Fig. 8, will be disclosed. It is noted that the disclosed technology is not limited to Examples below.

IPS cell clones A to O were prepared from peripheral blood mononuclear cells derived from different donors according to the method described in JP5984217B. mTeSR1 (Stem Cell Technologies) and Matrigel (Corning Inc.) were used for culturing iPS cells, and the iPS cells were subjected to expansion culture. The cells were recovered by treating with a 0.5 mM EDTA solution (Thermo Fisher Scientific, Inc.) for 7 minutes and subcultured, and at a time when the confluency reached about 80% in the T225 flask, the cells were recovered by being detached to be single cells with TrypLE Select (Thermo Fisher Scientific, Inc.), and the cell concentration was adjusted to $3.0 \times 10^6$ cells/ml in the mTeSR1 to which 1 $\mu$M H1152 (Fujifilm Wako Pure Chemical Industries, Ltd.), 25 $\mu$g/ml Gentamicin (Thermo Fisher Scientific, Inc.), and 100 ng/ml bFGF (Wako Pure Chemical Industries, Ltd.) had been added in terms of final concentration. 15 ml of the cell suspension was added to a single-use bioreactor (ABLE Corporation) having a capacity of 30 ml and subjected to spinner culture at a rotation speed of 40 rpm. After 2 to 4 hours from the start of the spinner culture, the cell suspension was adjusted to a final liquid volume of 30 ml in the same culture medium and continuously subjected to the spinner culture as it was.

1 day after the start of the spinner culture, the culture medium was exchanged to a culture medium consisting of 1 $\mu$M H1152, 25 $\mu$g/ml Gentamicin, 100 ng/ml bFGF, 24 ng/ml Activin, 5% fetal bovine serum (GE Healthcare), $\times$0.5 mTeSR1, and $\times$0.5 DMEM Low-glucose (Thermo Fisher Scientific, Inc.) in terms of final concentration, and then spinner culture was continued.

Two days after the start of the spinner culture, a part of the culture solution was sampled for each of the 15 clones, a hologram of the sphere was obtained using the image capturing system 1, and a phase contrast image of the obtained hologram of the sphere was generated.

The constitution of the image capturing system 1 used is as follows. As the laser beam source 11, a HeNe laser having a wavelength of 632.8 nm and an output of 5 mW was used. As the optical fiber 23, an optical fiber having NA = 0.11 was used. As the collimating lens 13, a collimating lens having f = 10 mm (NA = 0.4) was used. The diameter of the laser beam that is to the sphere was set to 2.2 mm. As the objective lens 15, an objective lens having NA = 0.45 and f = 20 mm was used. As the imaging lens 17, an imaging lens having f = 200 mm and an opening diameter of 36 mm was used, and light was imaged on the imaging surface of the CMOS camera at an image magnification of 10 times. As the collimating lens 21, a collimating lens having f = 100 mm (NA = 0.25) was used. As the CMOS camera 19, a monochrome image sensor having a resolution of 2,448 $\times$ 2,048 and a sensor size of 3.45 $\mu$m $\times$ 3.45 $\mu$m was used. The image capturing was carried out by tilting the beam splitter 18 so that the reference light is tilted by about 3° with respect to the object light, so that an off-axial optical system in which optical axis directions of the object light and the reference light, incident on the imaging surface of the CMOS camera 19, were different from each other.

After trimming the image acquired by the image capturing system 1 to a size of 2,048 $\times$ 2,048, a two-dimensional Fourier transform was carried out, and the complex amplitude component of only the object light was extracted by the frequency filtering process using a mask having a circular opening of a radius of 250 pixels. The angular spectral propagation method was used to restore the phase contrast image. The unweighted least squares method was used for phase unwrapping.

Subsequently, for each of the 15 clones, the phase contrast amount density Dp was derived using Expression (6) and Expression (7) from the phase contrast image. A part of the culture solution was sampled to measure the number of cells, and the culture solution was adjusted to $1.0 \times 10^6$ cells/ml in a culture medium consisting of 25 $\mu$g/ml Gentamaicin, 100 ng/ml bFGF, 24 ng/ml Activin, 40 ng/ml BMP4, 10% fetal bovine serum, and DMEM low-glucose in terms of final concentration, and then differentiation culture was started. The spinner culture was continued even during the differentiation culture. From 3 days to 7 days after the start of the spinner culture, the culture medium was changed daily with the same culture medium, and then the spinner culture was continued.

Eight days after the start of the spinner culture, a part of the culture solution was sampled to measure the number of cells, the culture solution was adjusted to $1.0 \times 10^6$ cells/ml in a culture medium consisting of 25 $\mu$g/ml Gentamaicin, 16.25 $\mu$g/ml XAV939, 10% fetal bovine serum, and DMEM low-glucose in terms of final concentration, and then spinner culture was continued. From 9 days to 13 days after the start of the spinner culture, the culture medium was changed every two days with the same culture medium excluding XAV939, and then spinner culture was continued.

14 days after the start of the spinner culture, it was confirmed that a part of cell masses derived from each of clones A, B, D, E, F, G, I, J, K, L, M, N, and O beat autonomously. On the other hand, the beating was not observed in the cell masses derived from clones C and H. A part of the culture solution was sampled to measure the number of cells. The number of cells finally obtained is shown in Table 1.

[Table 1]

| Clone name | Total number of cells ($\times 10^6$ cells) |
|---|---|
| A | 143.5 |
| B | 534.7 |
| C | 4.9 |
| D | 24.8 |
| E | 573.5 |
| F | 212.9 |
| G | 334.2 |
| H | 8.4 |
| I | 315.4 |
| J | 173.1 |
| K | 94.6 |
| L | 555.2 |
| M | 285.6 |
| N | 46.6 |
| O | 20.6 |

Cell masses 14 days after the start of the spinner culture were separated into single cells by TrypLE Select, and dead cells were stained using a Live/Dead Fixable Green Dead Cell Stain Kit. After washing with D-PBS (Thermo Fisher Scientific, Inc.), the cells were fixed by treatment with formaldehyde (Sigma-Aldrich Co., LLC) of a final concentration of 4%. An anti-cardiac Troponin T (cTnT) antibody (Abcam plc, ab8295) was diluted to 1/250 in the D-PBS containing 0.1% Saponin and 2% fetal bovine serum in terms of final concentration and added to $0.5 \times 10^6$ cells of the obtained fixed cells, and the treatment was carried out at room temperature for 1 hour. At the same time, as an isotype control, a sample to which IgG1 isotype Control Murine Myeloma (Sigma-Aldrich Co., LLC, M5284) diluted to 1/25 had been added were paralleled. Subsequently, Alexa647-labeled Goat anti-mouse IgG1 antibody (Thermo Fisher Scientific, Inc., A21240) was diluted to 1/500 and added, and the treatment was carried out at room temperature for 30 minutes. The obtained labeled cells were analyzed using a flow cytometer. After gating forward light scattering, lateral light scattering, and live cells, the cTnT positive cell rate was calculated by comparison with the Isotype sample. Table 2 shows the cTnT positive rate of each clone and the number of cTnT positive cells (total number of myocardial cells) calculated from the total number of cells and the cTnT positive rate.

[Table 2]

| Clone name | cTnT positive rate of cells (%) | Total number of myocardial cells ($\times 10^6$ cells) |
|---|---|---|
| A | 42.7 | 61.3 |
| B | 14.4 | 77.0 |
| C | 0.1 | 0.0 |
| D | 22.8 | 5.6 |
| E | 25.5 | 146.2 |
| F | 16.8 | 35.8 |
| G | 62.2 | 207.9 |
| H | 1.1 | 0.1 |

(continued)

| Clone name | cTnT positive rate of cells (%) | Total number of myocardial cells ($\times 10^6$ cells) |
| --- | --- | --- |
| I | 44.9 | 141.6 |
| J | 41.6 | 72.0 |
| K | 40.1 | 37.9 |
| L | 11.6 | 64.4 |
| M | 41.8 | 119.4 |
| N | 16.9 | 7.9 |
| O | 5.0 | 10.8 |

For each of the 15 clones, a proportion of the total number of myocardial cells to the number of cells at a time of the start of spinner culture was derived as the acquisition rate Y. Subsequently, the phase contrast amount density $D_p$ and the acquisition rate Y, acquired for every clone, were plotted on a graph, a graph that shows a relationship between the phase contrast quantity density Dp and the acquisition rate Y, shown in Fig. 8, was created.

Further, the relationship between the phase contrast amount density Dp and the acquisition rate Y was mathematically expressed using a linear combination model of the Gaussian function and the sigmoid function, which is a known function fitting method. Expression (9) could be derived as a function that shows a relationship between the phase contrast amount density Dp and the acquisition rate Y, shown in Fig. 8.

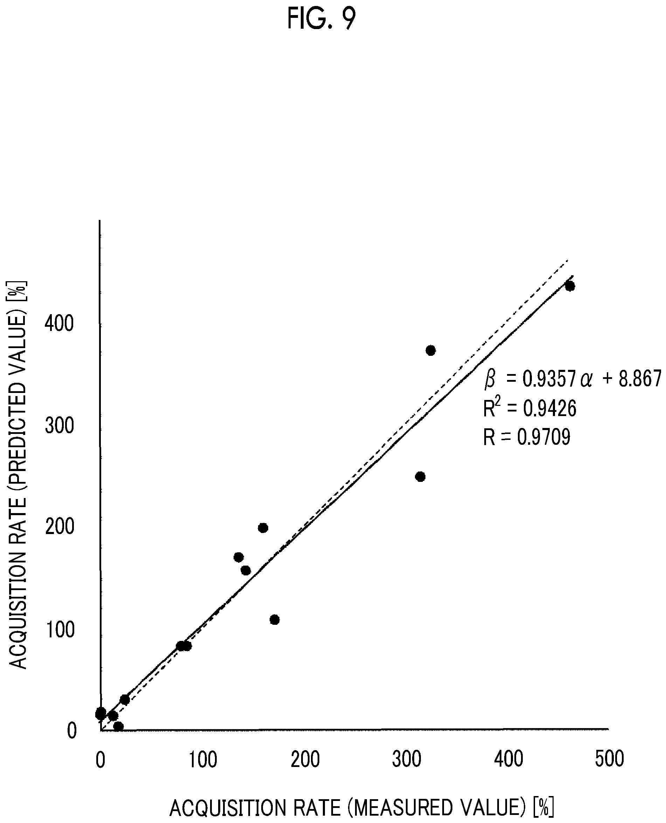

Fig. 9 shows a relationship between the predicted value (vertical axis) of the acquisition rate of myocardial cells calculated using Expression (9) and the measured value (horizontal axis) for each of the 15 clones. The predicted value and the measured value of the acquisition rate, shown by each plot in Fig. 9, are intra-lot average values. The dotted line in Fig. 9 is an ideal line in a case where the predicted value and the measured value coincide with each other, and the solid line in Fig. 9 is an approximate straight line derived based on each plot. In a case where the horizontal axis of the graph shown in Fig. 9 is denoted by $\alpha$ and the vertical axis thereof is denoted by $\beta$, the approximate straight line is expressed as $\beta = 0.9357a + 8.867$. The coefficient of determination $R^2$ in this approximate straight line is 0.9426, and the correlation coefficient R is 0.9709. That is, it can be said that the accuracy of the predicted value of the acquisition rate derived by using the function represented by Expression (9) is extremely high.

The disclosure of JP2019-195670 filed on October 28, 2019, is incorporated in the present specification in its entirety by reference. In addition, all documents, patent applications, and technical standards described in the present specification are incorporated in the present specification by reference, to the same extent as in the case where each of the documents, patent applications, and technical standards is specifically and individually described.

**Claims**

1. A sorting method for a pluripotent stem cell, comprising:

   generating a phase contrast image of an aggregate of a pluripotent stem cell from a hologram in which the aggregate is captured;
   deriving a phase contrast amount density obtained by dividing a total phase contrast amount which is a value obtained by integrating a phase contrast amount of each of a plurality of pixels that constitute the phase contrast image, by a volume of the aggregate; and
   sorting the pluripotent stem cell based on the phase contrast amount density.

2. The sorting method according to claim 1,
   wherein a pluripotent stem cell contained in an aggregate having the phase contrast amount density which is within a predetermined range is targeted for a differentiation induction treatment for differentiation into a specific cell.

3. The sorting method according to claim 2,
   wherein the predetermined range of the phase contrast amount density is determined based on a relationship between the phase contrast amount density acquired in regard to the aggregate before carrying out the differentiation induction treatment and an acquisition rate indicating a proportion of the number of the specific cells obtained by the differentiation induction treatment with respect to the number of pluripotent stem cells at a time before carrying out the differentiation induction treatment.

4. A prediction method for a differentiation induction result, comprising:

generating a phase contrast image of an aggregate of a pluripotent stem cell from a hologram in which the aggregate is captured;
deriving a phase contrast amount density obtained by dividing a total phase contrast amount which is a value obtained by integrating a phase contrast amount of each of a plurality of pixels that constitute the phase contrast image, by a volume of the aggregate; and
deriving, based on the phase contrast amount density, a predicted value regarding the number of specific cells obtained by carrying out a differentiation induction treatment for differentiating the pluripotent stem cell into the specific cell.

5. The prediction method according to claim 4,
wherein the predicted value is an acquisition rate indicating a proportion of the number of the specific cells obtained by the differentiation induction treatment with respect to the number of pluripotent stem cells at a time before carrying out the differentiation induction treatment.

6. The prediction method according to claim 5,
wherein a function that shows a relationship between the phase contrast amount density and the acquisition rate is used to derive the predicted value.

7. A production method for a cell product, comprising:

a culture step of culturing a pluripotent stem cell;
a sorting step of sorting the pluripotent stem cell cultured in the culture step; and
a differentiation induction step of carrying out a differentiation induction treatment for differentiating the pluripotent stem cell sorted in the sorting step into a specific cell,
wherein in the sorting step,
a phase contrast image of an aggregate of the pluripotent stem cell is generated from a hologram in which the aggregate is captured,
a phase contrast amount density obtained by dividing a total phase contrast amount which is a value obtained by integrating a phase contrast amount of each of a plurality of pixels that constitute the phase contrast image is derived, by a volume of the aggregate, and
the pluripotent stem cell is sorted based on the phase contrast amount density.

8. The method according to any one of claims 2 to 7,
wherein the specific cell is a myocardial cell.

9. The method according to any one of claims 2 to 8,
wherein the phase contrast amount density is derived based on the phase contrast image generated from the hologram in which the aggregate before carrying out the differentiation induction treatment is captured.

## FIG. 1

| EXPANSION CULTURE STEP | ~S1 |

| THREE-DIMENSIONAL CULTURE STEP | ~S2 |

| SORTING STEP | ~S3 |

| DIFFERENTIATION INDUCTION STEP | ~S4 |

## FIG. 2

| PHASE CONTRAST IMAGE IS GENERATED FROM HOLOGRAM IN WHICH SPHERES ARE CAPTURED | ~S11 |

| PHASE CONTRAST AMOUNT DENSITY $D_P$ OF SPHERES IS DERIVED | ~S12 |

| PLURIPOTENT STEM CELLS ARE SORTED BASED ON PHASE CONTRAST AMOUNT DENSITY $D_P$ | ~S13 |

# FIG. 3

FIG. 4A

FIG. 4B

## FIG. 4C

## FIG. 4D

# FIG. 5

FIG. 6

STATE OF BEING IN FOCUS

STATE OF BEING OUT OF FOCUS

STATE OF BEING OUT OF FOCUS

STATE OF BEING IN FOCUS

PHASE CONTRAST AMOUNT

CONTRAST AMOUNT

FREQUENCY

PHASE CONTRAST AMOUNT

POSITION

# FIG. 7

# FIG. 8

# FIG. 9

$\beta = 0.9357\,\alpha + 8.867$
$R^2 = 0.9426$
$R = 0.9709$

ACQUISITION RATE (PREDICTED VALUE) [%]

ACQUISITION RATE (MEASURED VALUE) [%]

# INTERNATIONAL SEARCH REPORT

International application No.

PCT/JP2020/037590

### A. CLASSIFICATION OF SUBJECT MATTER
Int.Cl. G01N21/17(2006.01)i, C12N5/071(2010.01)i, C12Q1/04(2006.01)i
FI: C12Q1/04, C12N5/071, G01N21/17A

According to International Patent Classification (IPC) or to both national classification and IPC

### B. FIELDS SEARCHED
Minimum documentation searched (classification system followed by classification symbols)
Int.Cl. G01N21/17, C12N5/071, C12Q1/04

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

### C. DOCUMENTS CONSIDERED TO BE RELEVANT

| Category* | Citation of document, with indication, where appropriate, of the relevant passages | Relevant to claim No. |
|---|---|---|
| X | WO 2019/176427 A1 (FUJIFILM CORPORATION) 19 September 2019 (2019-09-19), claims, examples, paragraph [0088] | 1-9 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 October 2020 | 10 November 2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

| INTERNATIONAL SEARCH REPORT | International application No. |
|---|---|
| Information on patent family members | PCT/JP2020/037590 |

WO 2019/176427 A1  19 September 2019    (Family: none)

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015146747 A
- JP 2018000048 A
- WO 2018158901 A
- JP 5984217 B
- JP 2019195670 A